# EUROPEAN PATENT SPECIFICATION

(11) **EP 1 933 642 B1**
(45) Date of publication and mention of the grant of the patent: **21.12.2011**
(21) Application number: 06806185.2
(22) Date of filing: 11.10.2006
(51) Int. Cl.: A23L 1/227, A23L 1/237

(54) **SALT ENHANCING COMPOUNDS AND USE**
SALZVERSTÄRKENDE VERBINDUNGEN UND IHRE VERWENDUNG
COMPOSES EXHAUSTEURS DE GOUT SALE ET UTILISATION DE CEUX-CI

(30) Priority: 14.10.2005 GB 0520845
(43) Date of publication of application: 25.06.2008
(73) Proprietor: Hofmann, Thomas Frank, 85375 Neufahrn (DE); Dunkel, Andreas, 85354 Freising (DE)
(72) Inventor: HOFMANN, Thomas, Frank, 85375 Neufahrm (DE); DUNKEL, Andreas, 85354 Freising (DE)
(74) Representative: Givaudan Patents
(86) International application number: PCT/EP2006/009818
(87) International publication number: WO 2007/042274

(56) References cited:
- EP-A- 1 252 825
- EP-A1- 0 827 967
- EP-B1- 0 181 421
- US-A- 5 145 707
- US-A- 5 780 090

## Description

This invention relates to the use of salt enhancing compounds in consumables, to flavour compositions comprising such compounds and to novel compounds with salt-enhancing properties.

Saltiness is one of the basic taste qualities (salty, sweet, bitter, sour and umami), and can easily be detected and differentiated with sensory tests by a trained panel, Saltiness is perceived upon tasting sodium and/or potassium chloride (NaCl/KCl). To provide or enhance a salty taste is of particular interest in the flavour industry, as It improves the overall flavour of a consumable. However, a high amount of sodium intake is considered to be detrimental to health. To provide a salty taste to consumables, usually sodium in form of sodium chloride (NaCl, ordinary table salt) is used. NaCl can be partly replaced by potassium chloride (KCl), however, KCI has an unpleasant bitter/metallic off-taste hence only about 20% NaCl can be replaced by KCI.

Therefore there is a desire for salt-enhancing compounds that will increase the salty taste at a given salt concentration, or alternatively will allow the reduction of the amount of salt without reducing the desired taste at the same time. The salty taste is very important to the perceived flavour intensity and profile, especially for savoury food products, and In smaller concentrations also for non-savoury applications, for example fruit juices, or desserts.

Various compounds that enhance the saltiness are known. US 2005/0123670 discloses compositions comprising various mineral salts including KCl, magnesium salts and acids to replace NaCl. WO 2003/022817 discloses pyridinium-betain compounds. However, the synthesis and purification of these compounds is difficult US 6,159,529 discloses a certain sugar compound (trehalose) that enhances the salty taste of NaCl when used in high concentrations of 1.5-12%. As it is a sugar, a sweet taste will be introduced at the same time. A salt-enhancing composition comprising L-aspartic acid and L-arginine and sodium chloride Is known from US 5,145,707. The combination of L-aspartic acid with L-arginine has the ability to enhance the salty flavour of NaCl in foods without sour or bitter taste.

EP181421 discloses sulphur containing compounds as flavour enhancers for foods.

Various mixtures of the salt forms of amino acids and peptides have been proposed as salt substitutes, for example, the monohydrochloride salts of the basic dipeptides omithyltaurine and ornithyl-beta-alanine, and a composition comprising glutamic acid and aspartic acid either in acid form or as non-sodium salts. US 2,500,919 describes a sodium-free seasoning composition capable of yielding monopotassium glutamate In the presence of water as a meat flavouring agent, US 4,340,614 desribes a salt substitute consisting of KCl mixed with the potassium salts of adipic, tartaric, and glutamic acid plus a 5'-nucleotide, A chloride-free product with a saline taste is disclosed in US 1,874,055 and comprises the alkali metal salt of formic, acetic or lactic acid with glutamic acid or a protein hydrolysate capable of yielding mixtures of different amino acids. Tamura et al. (see Agric. Biol. Chem., 53(6):1625-33, 1989) have shown that the saltiness of NaCl in aqueous solution can be modified by the addition of the hydrochloride salts of certain amino compounds (amino acids and amino acid esters). Both enhancement of salty flavour and diminishment of salty flavour were observed when the amino compound was present at concentrations ranging from 0.0075 to 0.045 mol/L.

Attempts to provide sodium-free or low sodium salt substitutes or enhancers have been only partially successful, as the proposed compositions have a low saltiness value when compared to NaCl, provide undesirable off-tastes perceived as unpleasant by the consumer, or otherwise do not provide a flavour quality equal to that of NaCl. In many cases, undesirable off-flavours or undesired flavour notes (bitter, sour, umami, sweet) are introduced. While umami, sweet and sour may be useful in some applications, bitter notes or metallic off-tastes are generally found undesirable by consumers in most consumables.

Further, some salt substitutes or enhancers are difficult to produce or extremely expensive, especially when compared to ordinary table salt.

There remains a need for alternative or improved compounds and compositions to provide or enhance the saltiness in consumables without introducing unpleasant off-tastes (in particular bitter or metallic).

In addition, these compounds and compositions preferably should be inexpensive to produce and stable during long periods of storage and to processing conditions that may comprise elevated temperatures and humidity, and extremes of pH.

Surprisingly, applicant has identified a group of compounds according to formula I hereinunder that are useful for increasing the saltiness in consumables comprising salt, and that fulfill the abovementioned requirements. Advantageously, all of compounds of formula I have a very good oxidative stability in consumables and in the presence of air.

In a first aspect, the invention is therefore directed to the use of a compound of formula I, or a salt thereof, as a flavour or salt enhancer, 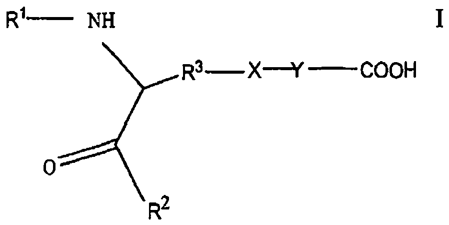 wherein the residues R¹, R², R³, X and Y are selected as follows:
R¹ is a residue selected from the group of H, an amino acid linked via a peptide bond selected from the group consisting of γ-Glu, α-Glu, β-Asp, α-Asp, β-Ala, α-Ala, α-Val, α-Leu, α-Ile, α-Met, α-Pro, α-Phe, α-Trp, α-Ser, α-Thr, α-Asn, α-Gln, α-Tyr, α-Cys, α-Lys, α-Arg, α-His, α-Asp, α-Glu, gamma amino butyric acid (GABA), and an uncommon amino acid including 4-hydroxyprolin, ε-N,N,N-trimethyllysine, 3-methylhistidine, 5-hydroxylysine, O-phosphoserine, gamma-carboxyglutamate, ε-N-acetyllysine, ω-N-methylarginine, N-acetylserine, N,N,N-trimethylalanine, N-formylmethionine;
R² is a residue selected from the group consisting of OH, a C₁-C₅ linear or branched alkoxy residue including -O-CH₃, -O-CH₂-CH₃, -O-CH₂CH₂CH₃, -O-CH(CH₃)CH₃, -O-CH₂CH(CH₃)₂, -O-CH₂CH(CH₃)(CH₂CH₃), and -O-CH₂CH₂CH(CH₃)₂, and an amino acid linked via a peptide bond selected from the group consisting of Gly, β Ala, α-Ala, α-Val, α-Leu, α-Ile, α-Met, α-Pro, α-Phe, α-Trp, α-Ser, α-Thr, α-Asn, α-Gln, α-Tyr, α-Cys, α-Lys, α-Arg, α-His, α-Asp, α-Glu, , gamma amino butyric acid (GABA), and an uncommon amino acid including 4-hydroxyprolin, ε-N,N,N-trimethyllysine, 3-methylhistindine, 5-hydroxylysine, O-phosphoserine, gamma-carboxyglutamate, ε-N-acetyllysine, ω-N-methylarginine, N-acetylserine, N,N,N-trimethylalanine, N-formylmethionine;
R³ is a residue selected from -CH₂- or -CH₂CH₂-;
X is a residue selected from -S- or -0-; and
Y is a residue selected from the group consisting of CH(COOH)-, -C(COOH)₂-, -C(CH₂COOH)₂-, -C(CH₂COOH)(COOH)-, -C(CH(COOH)₂)(COOH)-, -CH(CH₂COOH)-, -CH(CH₂CH₂COOH)-, -CH(CH₂CH₂CH₂COOH)-, -CH(CH(COOH)₂)-, -CH(CH(COOH)CH(COOH)₂)-, - CH(CH₂CH(COOH)₂)-, -CH(CH(COOH)CH₂COOH)-, -CH₂-CH(COOH)-, -CH₂-C(COOH)₂-, -CH₂-C(CH₂COOH)₂-, -CH₂-C(CH₂COOH)(COOH)-, -CH₂-C(CH(COOH)₂)(COOH)-, -CH₂-CH(CH₂COOH)-, -CH₂-CH(CH₂CH₂COOH)-, -CH₂-CH(CH₂CH₂CH₂COOH)-, -CH₂-CH(CH(COOH)₂)-, -CH₂-CH(CH(COOH)CH(COOH)₂)-, -CH₂-CH(CH₂CH(COOH)₂)-, -CH₂-CH(CH(COOH)CH₂COOH)-, -CH(COOH)-CH₂-, -C(COOH)₂-CH₂-, -C(CH₂COOH)₂-CH₂-, -C(CH₂COOH)(COOH)-CH₂-, -C(CH(COOH)₂)(COOH)-CH₂-, -CH(CH₂COOH)-CH₂-, -CH(CH₂CH₂COOH)-CH₂-, -CH(CH₂Ch₂CH₂COOH)-CH₂-, -CH(CH(COOH)₂)-CH₂-, - CH(CH(COOH)CH(COOH)₂)-CH₂-, -CH(CH₂CH(COOH)₂-)-CH₂-, -CH(CH(COOH)CH₂COOH)-CH₂-.

Residues R¹ and R² are linked to formula I (NH or CO of formula I, respectively) via a peptide bond, for example, for R¹: γ-Glu (-CO-CH₂-CH₂-CH(NH₂)-COOH), α-Glu (-CO-CH(NH₂)-CH₂-CH₂-COOH), β-Asp (-CO-CH₂-CH(NH₂)-COOH), α-Asp (-CO-CH(NH₂)-CH₂-COOH), β-Ala (-CO- CH₂- CH₂-NH₂), α-Ala (-CO-CH(CH₃)-NH₂); and for R²: Gly (NH-CH₂COOH), β-Ala (-NH-CH₂- CH₂-COOH), α-Ala (-NH-CH(CH₃)-COOH).

An "uncommon" or nonstandard amino acid is a derivative of one of the 20 standard amino acids that occur in biological systems; some occur as components of proteins occurring in nature, some are biologically active peptides.

A compound of formula I may be present in the form as shown or in its ionic form with or without a counter-ion (in form of its salt), for example its sodium, potassium, calcium, ammonium, chloride, sulfate, phosphate, carbonate salt, or similar counter-ion. While the use of a salt enhancer compound in the form of its sodium salt will still significantly reduce the overall sodium content in the composition or consumable, the use of a non-sodium counter-ion will further minimise the sodium content.

In addition to the salt enhancement, compounds according to Formula I wherein R¹ is γ-Glu or β-Asp also provide a kokumi-taste to consumables. "Kokumi" is a term used in the flavour industry to describe characteristics such as continuity, mouthfulness, richness and thickness. In contrast thereto, the sensory terms for the basic tastes are salty, sweet, sour, bitter or umami, the latter being the taste of monosodium glutamate (MSG). Kokumi is a distinct taste quality, or rather taste enhancing quality, which can be easily be detected and differentiated with sensory tests by a trained panel. Compounds that provide a kokumi taste enhance the taste in combination with other tastants in respect of the above-mentioned qualities.

In another aspect, compounds selected from compounds according to formula I wherein R² is a residue selected from the group consisting of OH, Gly, and β-Ala.

In another aspect, compounds selected from compounds according to formula I wherein R² is a C₁-C₅ linear or branched alkoxy residue including -O-CH₃, -O-CH₂-CH₃, -O-CH₂CH₂CH₃, -O-CH(CH₃)CH₃, -O-CH₂CH(CH₃)₂, -O-CH₂CH(CH₃)(CH₂CH₃), and -O-CH₂CH₂CH(CH₃)₂.

In another aspect, the invention is directed to the use of a group of compounds wherein R¹ is γ-Glu, R² is a residue selected from the group consisting of OH, Gly, and β-Ala, and R³ is a residue selected from CH₂ and CH₂CH_{2;} X is a residue selected from S and O; and Y is a residue selected from the group consisting of CH(COOH), CH(COOH)CH₂, and CH(CH₂COOH)CH₂, CH(CH₂CH₂COOH). In a particular embodiment, the invention is directed to the use of the group of compounds as hereinabove defined wherein X is S. In another embodiment, the invention is directed to the use of the group of compounds as hereinabove defined wherein R³ is CH₂.

In another aspect, the invention is directed to the use of a group of compounds wherein R¹ is γ-Glu, R₂ is a C₁-C₅ linear or branched alkoxy residue including -O-CH₃, -O-CH₂-CH₃, -O-CH₂CH₂CH₃, -O-CH(CH₃)CH₃, -O-CH₂CH(CH₃)₂, -O-CH₂CH(CH₃)(CH₂CH₃), and -O-CH₂CH₂CH(CH₃)₂, and R³ is a residue selected from CH₂ and CH₂CH₂, X is a residue selected from S and O; and Y is a residue selected from the group consisting of CH(COOH), CH(COOH)CH₂, and CH(CH₂COOH)CH₂, CH(CH₂CH₂COOH). In a particular embodiment, the invention is directed to the use of the group of compounds as hereinabove defined wherein X is S. In another embodiment, the invention is directed to the use of the group of compounds as hereinabove defined wherein R³ is CH₂.

Examples of subgroups of these useful groups are listed below with residues R¹-R³, X and Y. These provide a very good salt enhancement activity.

**Table 1 Examples of compound groups according to formula I with residues as shown**

| | **R¹** | **R²** | **R3** | **X** | **Y** |
|---|---|---|---|---|---|
| 1 | γ-Glu | OH, Gly, βAla, OCH₃, OC₂H₅ | CH₂ | S | CH(COOH) |
| 2 | γ-Glu | OH, Gly, βAla, OCH₃, OC₂H₅ | CH₂CH₂ | S | CH(COOH) |
| 3 | γ-Glu | OH, Gly, βAla, OCH₃, OC₂H₅ | CH₂ | S | CH(COOH)CH₂ |
| 4 | γ-Glu | OH, Gly, βAla, OCH₃, OC₂H₅ | CH₂CH₂ | S | CH(COOH)CH₂ |
| 5 | γ-Glu | OH, Gly, βAla, OCH₃, OC₂H₅ | CH₂ | S | CH(CH₂COOH)CH₂ |
| 6 | γ-Glu | OH, Gly, βAla, OCH₃, OC₂H₅ | CH₂CH₂ | S | CH(CH₂COOH)CH₂ |
| 7 | γ-Glu | OH, Gly, βAla, OCH₃, OC₂H₅ | CH₂ | S | CH(CH₂CH₂COOH) |
| 8 | γ-Glu | OH, Gly, βAla, OCH₃, OC₂H₅ | CH₂CH₂ | S | CH(CH₂CH₂COOH) |
| 9 | γ-Glu | OH, Gly, βAla, OCH₃, OC₂H₅ | CH₂ | O | CH(COOH) |
| 10 | γ-Glu | OH, Gly, βAla, OCH₃, OC₂H₃ | CH₂CH₂ | O | CH(COOH) |
| 11 | γ-Glu | OH, Gly, βAla, OCH₃, OC₂H₅ | CH₂ | O | CH(COOH)CH₂ |
| 12 | γ-Glu | OH, Gly, βAla, OCH₃, OC₂H₅ | CH₂CH₂ | O | CH(COOH)CH₂ |
| 13 | γ-Glu | OH, Gly, βAla, OCH₃, OC₂H₅ | CH₂ | O | CH(CH₂COOH)CH₂ |
| 14 | γ-Glu | OH, Gly, βAla, OCH₃, OC₂H₅ | CH₂CH₂ | O | CH(CH₂COOH)CH₂ |
| 15 | γ-Glu | OH, Gly, βAla, OCH₃, OC₂H₅ | CH₂ | O | CH(CH₂CH₂COOH) |
| 16 | γ-Glu | OH, Gly, βAla, OCH₃, OC₂H₅ | CH₂CH₂ | O | CH(CH₂CH₂COOH) |

In another aspect, the invention is directed to the use of compounds as hereinabove defined wherein X is S.

In another aspect, the invention is directed to the use of compounds as hereinabove defined wherein R³ is CH₂.

In another aspect, the invention is directed to the use of compounds as hereinabove defined wherein Y is selected from the group consisting of CH(COOH), CH(COOH)CH₂, CH(CH₂COOH)CH₂, and CH(CH₂CH₂COOH).

In another aspect, the invention is directed to the use of compounds as hereinabove defined Y is selected from the group consisting of CH(COOH)-, -CH(COOH)CH₂-, -CH(CH(COOH)₂)-, -CH₂CH₂-, -CH₂CH₂ CH₂-, and - CH(CH₂COOH)CH₂.

Some of the above mentioned compounds are novel. Therefore, in another of its aspects, the invention is directed to a compound of the formula I as shown hereinabove with the proviso that the compound is not selected from the group consisting of S-(α,β-dicarboxyethyl) γ-L-glutamyl-L-cysteinyl-glycine, S-(α,β-dicarboxyethyl) cysteine, 3-(carboxymethoxy)-alanine, S-carboxymethyl-glutathione (glutaramic acid), S-carboxymethyl-cysteinyl-glycine, (S-carboxymethyl)-lysyl-cysteine, S-dicarboxymethyl-glutathione, S-carboxymethyl-cysteine, S-(1,2-dicarboxyethyl)-glutathione, and S-(1,2-dicarboxyethyl)-cysteine.

The first two of the above-mentioned compounds have been previously reported as a pharmaceutical and were detected in yeast (Tsuboi, S. et al., Biological & Pharmaceutical Bulletin, 1999, 22, 21-25) and in animal tissues including ox liver (Azumi T., Acta. Med. Okayama, 1967, 121, 316-320 and 321-326). Other compounds are known as reaction intermediates. However, the use as a salt enhancer or flavour has not been suggested previously.

In another aspect the invention is directed to a method to form a γ-glutamyl dipeptide compound of formula I as defined in any one of claims 1 to 10 by chemical or enzymatical synthesis , using γ-glutamyl-transpeptidase.

In a particular embodiment, compounds are selected from the group consisting of S-(α,β-dicarboxyethyl) γ-L-glutamyl-cysteine, β-S-(carboxyethyl) γ-L-glutamyl-L-cysteinyl-glycine, ß-S-(α,γ-dicarboxypropyl) γ-L-glutamyl-L-cysteinyl-glycine, S-(α,β-dicarboxyethyl) ß-L-asparagyl-L-cysteinyl-glycine, S-(α,β-dicarboxyethyl) ß-L-asparagyl-cysteine, S-(α,β-dicarboxyethyl) γ-L-glutamyl-L-cysteinyl-beta-alanine), S-(α,β-dicarboxyethyl) ß-L-asparagyl-L-cysteinyl-beta-alanine, α-S-(α,γ-dicarboxypropyl) γ-L-glutamyl-L-cysteinyl-glycine, α-S-(α,γ-dicarboxypropyl) γ-L-glutamyl-L-cysteine, α-S-(α,γ-dicarboxypropyl) L-cysteine, ß-S-(α,γ-dicarboxypropyl) γ-L-glutamyl-L-cysteine, and ß-S-(α,γ-dicarboxypropyl) L-cysteine.

Some particular examples of compounds for use as a flavour or salt enhancer according to formula I with its residues with residues R¹-R³, X and Y are shown in the table below

**Table 2 Example compounds according to formula I with residues as shown.**

| **compound** | **R¹** | **R²** | **R3** | **X** | **Y** |
|---|---|---|---|---|---|
| S-(α,β-dicarboxyethyl) γ-L-glutamyl-L-cysteinyl-glycine | -CO-CH₂-CH₂-CH(NH₂)-COOH | NH-CH₂COOH | CH₂ | S | CH(COOH)CH₂ |
| S-(α,β-dicarboxyethyl) γ-L-glutamyl-cysteine | -CO-CH2-CH₂-CH(NH₂)-COOH | OH | CH₂ | S | CH(COOH) CH₂ |
| S-(α,β-dicarboxyethyl) γ-L-glutamyl-cysteine methyl ester | -CO-CH₂-CH₂-CH(NH₂)-COOH | OCH₃ | CH₂ | S | CH(COOH) CH₂ |
| S-(α,β-dicarboxyethyl) γ-L-glutamyl-cysteine ethyl ester | -CO-CH₂-CH₂-CH(NH₂)-COOH | OCH₂CH₃ | CH₂ | S | CH(COOH) CH₂ |
| S-(α,β -dicarboxyethyl) cysteine | H | OH | CH₂ | S | CH(COOH)CH₂ |
| S-(α,β -dicarboxyethyl) cysteine methyl ester | H | OCH₃ | CH₂ | S | CH(COOH)CH₂ |
| S-(α,β -dicarboxyethyl) cysteine ethyl ester | H | OCH₂CH₃ | CH₂ | S | CH(COOH)CH₂ |
| β-S-(α,γ-dicarboxypropyl) γ-L-glutamyl-L-cysteinyl-glycine | -CO-CH₂-CH₂-CH(NH₂)-COOH | NH-CH₂COOH | CH₂ | S | CH (CH₂COOH)CH₂ |
| S-(α,β-dicarboxyethyl) β-L-asparagyl-L-cysteinyl-glycine | -CO-CH₂-CH(NH₂)-COOH | NH-CH₂COOH | CH₂ | S | CH(COOH) CH₂ |
| S-(α,β-dicarboxyethyl) β-L-asparagyl-cysteine | -CO-CH₂-CH(NH₂)-COOH | OH | CH₂ | S | CH(COOH) CH₂ |
| S-(α,β-dicarboxyethyl) γ-L-glutamyl-L-cysteinyl- β -alanine) | -CO-CH₂-CH₂-CH(NH₂)-COOH | β -Ala | CH₂ | S | CH(COOH) CH₂ |
| S-(α,β-dicarboxyethyl) β-L-asparagyl-L-cysteinyl- β -alanine | -CO-CH₂-CH(NH₂)-COOH | β-Ala | CH₂ | S | CH(COOH) CH₂ |
| α-S-(α,γ-dicarboxypropyl) γ-L-glutamyl-L-cysteinyl-glycine | -CO-CH₂-CH(NH₂)-COOH | NH-CH₂COOH | CH₂ | S | CH(CH₂ CH₂COOH) CH₂ |
| α-S-(α,γ-dicarboxypropyl) γ-L-glutamyl-L-cysteine | -CO-CH₂-CH(NH₂)-COOH | OH | CH₂ | S | CH(CH₂ CH₂COOH) CH₂ |
| α-S-(α,γ-dicarboxypropyl) L-cysteine | H | OH | CH₂ | S | CH(CH₂CH₂COOH)CH₂ |
| β-S-(α,γ-dicarboxypropyl) γ-L-glutamyl-L-cysteine | -CO-CH₂-CH₂-CH(NH₂)-COOH | OH | CH₂ | S | CH (CH₂COOH)CH₂ |
| β-S-(α,γ-dicarboxypropyl) L-cysteine | H | OH | CH₂ | S | CH (CH₂COOH)CH₂ |

In another aspect, the invention is directed to a flavour composition comprising at least one compound of formula I as defined hereinabove. A flavour composition may comprise excipients commonly known in the art. In a particular embodiment, the invention is directed to a composition comprising a compound of formula I as defined hereinabove wherein the compounds are added in form of a crude or purified extract selected from the group consisting of a enzyme extract, a plant extract, a fermentation extract, a cell culture fermentation extract, a bacteria fermentation extract, a fungi fermentation extract, and a yeast fermentation extract

In another aspect, the invention is directed to consumables comprising a compound of formula (I) as defined hereinabove, or mixtures thereof. The compound may be present in a concentration of 1 to 10.000 ppm.

In another aspect, the invention is directed to a method for enhancing the saltiness of a consumable comprising the addition of a compound as defined hereinabove, or a composition defined hereinabove comprising a compound as defined hereinabove, and an amount of salt of at least 5 mmol/kg.

In another aspect, the invention provides a method for enhancing the saltiness of a consumable, comprising the addition of a compound as hereinabove described to a salt-containing consumable. To enhance the saltiness of a given consumable, the consumable should contain an amount of salt, for example, at least 5 mmol/kg, 10 mmol/kg, 20 mmol/kg, 30 mmol/kg or 40 mmol/kg NaCl.

In a use according to the invention, compounds as hereinabove defined may be advantageoulsy combined with a compound selected from the group consisting of arginine aspartate (decribed in US 5,145,707) and arginine formate, to provide an even more intensive salty taste without off-taste (metallic note). While arginine aspartate or formate when used without a compound of formula I will provide a metallic note, in combination this is significantly less pronounced or absent, depending on the concentration. The inventive composition may comprise a mixture of a compound of formula I as hereinabove defined and a compound listed above in a ratio of 10:1 to 1:10. Useful concentration ratios include, for example, 5:1 to 1:5, 2:1 to 1:2, 1.5:1 to 1.5:1, and 1.2:1 to 1:1.2.

A well working composition comprises, for example, a compound of formula I selected from compounds of table 1 or 2 and a compound selected from the group consisting of arginine aspartate and arginine formate.

Accordingly, in another aspect, the invention is directed to a composition comprising a compound of formula I as hereinabove defined and a compound selected from the group consisting of arginine aspartate and arginine formate.

In another aspect, the invention is directed to a method of forming novel compounds of formula I by enzymatical synthesis using gamma-glutamyl-transpeptidase, with the proviso that they are not selected from the group S-(α,β-dicarboxyethyl) γ-L-glutamyl-L-cysteinyl-glycine, S-(α,β-dicarboxyethyl) cysteine, 3-(carboxymethoxy)-alanine, S-carboxymethyl-glutathione (glutaramic acid), S-carboxymethyl-cysteinyl-glycine, (S-carboxymethyl)-lysyl-cysteine, S-dicarboxymethyl-glutathione, S-carboxymethyl-cysteine, S-(1,2-dicarboxyethyl)-glutathione, and S-(1,2-dicarboxyethyl)-cysteine.

Compounds for use in the present invention may be prepared according to procedures known in the art. One possibility is the chemical synthesis of peptides from amino acids which is well-known in the art. Non-natural amino acids can be formed by introducing side chains as desired and this also is well-known in the art.

For example, the reaction between a thiol compound, e.g. cysteine (H-Cys-OH), γ-L-glutamyl-L-cysteinyl-glycine (H-γ-Glu-Cys-Gly-OH), γ-L-glutamyl-cysteine (H-γ-Glu-Cys-OH), ß-L-asparagyl-L-cysteinyl-glycine (H-ß-Asp-Cys-Gly-OH), ß-L-asparagyl-cysteine (H-ß-Asp-Cys-OH), and an unsaturated carbonyl compound, having at least one double bond (e.g. maleic acid, glutaconic acid, acrylic acid, acetonitic acid, fumaric acid, 2-pentenoic acid) is performed according to the procedure reported by Morgan and Friedmann (Biochemical Journal; 32 (1938); 733-742). The unsaturated carbonyl (10 mmol) and the thiol compound (10 mmol) are dissolved in water (100 ml), adjusted to pH 7.4 with NaOH (1 mol/L) and incubated at 37°C for 24 h. After freeze-drying, the target compounds are purified by means of gel permeation chromatography using Sephadex G-10 (Amersham Bioscience, Uppsalla, Sweden) as stationary phase and water as mobile phase. The target compound elute after 800 mL of mobile phase, the identity and purity is checked by means of LC-MS and ¹H NMR spectroscopy.

Certain γ-glutamyl dipeptides of formula I can be prepared enzymatically using gamma-glutamyl-transpeptidase enzyme (GGTP) as is well known in the art using enzymes from various sources including commercial sources and described previously, for example, by Suzuki et al., J. Mol. Catal. 1999, B6, 175-184; Suzuki et al., J. Agric. Food Chem. 2002, 50, 313-318), Suzuki et al.; J. Agric. Food Chem.; 52 (2004); 577-580; Strumeyer and Bloch, Biochem. Prep.1962, 9, 52-55; Thompson and Meister, Proc. Nat. Acad. Sci. USA,1975, 72,1985-1988; Allison and Meister, J. Biol. Chem. 1981, 256, 2988-2992; Meister, The Enzymes B(Academi, New York), 3rd. Ed., Vol.10, pp. 671-697; Strumeyer and Bloch, J. Biol. Chem.1969, 235, 27; Thompson and Meister, Proc. Nat. Acad. Sci. USA, 1975, 72, 1985-1988; Oppenheimer et al., J. Biol. Chem.1979, 254, 5184-5190; Tate and Meister, J. Biol. Chem., 1975, 250, 4619-4627.

Starting materials and the enzymes are readily available commercially or can be obtained as described in the references indicated above.

S-(α,β -dicarboxyethyl) γ-L-glutamyl-L-cysteinyl-glycine may also be formed in yeast as described by Tsuboi, S. et al, Biological & Pharmaceutical Bulletin, 1999, 22, p. 21-25. Alternatively the enzyme may be first purified and the enzymatic reaction perfomed subsequently.

The formed products may be purified and used as a flavour in purified form, or they may be used as a flavour in crude form (enzymatic reaction mixture) or as a crude extract from fermentation or from enzymatic reaction with the isolated enzyme.

Compounds according to formula I provide salt-enhancing properties in consumables. By salt, NaCl and or KCI, or the corresponding dissociated ions are meant. The compounds of formula I enhance the salt taste whereby the taste of the salt that is present is rendered more noticeable, and either the consumable tastes more salty, or the salt concentration can be reduced to provide the same degree of saltiness (isosaltiness) at a reduced NaCl and/or KCI concentration.

The degree of enhancement in consumables may be, for example, an additional 25% up to 100% (saltiness is doubled), or 200% or more. Accordingly, it is possible to reduce the sodium content by more than half (+100%) and achieve the same saltiness (or even more if KCI is employed).

The degree of salt enhancement may be determined by isosaltiness to NaCl solutions compared in triangle tests as described in the examples at a given salt enhancer concentration, for example when the salt enhancing compounds are used at a concentration of about 10 mmol/L (about 300-350 ppm for most compounds, depending on molecular weight).

An appropriate concentration in which to employ compounds will depend on the type of consumable and the desired flavour intensity. For example, compounds according to the invention may be employed at a concentration of, for example, 1 to 10.000 ppm, 5 to 25.000 ppm, 10 to 10.000 ppm, 50 to 5000 ppm, and 100 to 1000 ppm (based on weight).

Consumables as used herein include food products, beverages, oral care products, and compositions for admixture to such products, in particular flavour compositions. Flavour compositions may be added to processed foods or beverages during their processing, or they may actually be consumables in their own right, e.g. condiments such as sauces and the like.

A compound of the present invention or a mixture thereof may be used as a flavour ingredient in flavour compositions. A compound or mixture of compounds may be blended with other flavour ingredients in said compositions. A compound or mixture of compounds enhances saltiness in all kinds of consumables, and is particularly interesting in savoury consumables.

Examples of consumables include cereal products, baker's products, bread products, gums, chewing gums, yeast products, salt and spice products, mustard products, vinegar products, sauces (condiments), soups, processed foods, cooked fruits and vegetable products, meat and meat products, egg products, milk and dairy products, cheese products, butter and butter substitute products, milk substitute products, soy products, edible oils and fat products, medicaments, beverages, alcoholic drinks, beers, soft drinks, food extracts, plant extracts, meat extracts, condiments, sweeteners, nutraceuticals, pharmaceutical and non-pharmaceutical gums, tablets, lozenges, drops, emulsions, elixirs, syrups and other preparations for making beverages, instant beverages and effervescent tablets.

By addition of compounds according to the invention, consumables moderate, reduced or low in sodium or salt may be formed (sodium concentrations are given below. To calculate salt content, multiply by 2.5). 250 mg to 1250 mg per 100 g or ml sodium is usually considered a moderate amount, while consumables above 1250 mg per 100 g or ml is considered a high amount. 0 to 250 mg / 100 g or ml can be considered a low amount.

Consumables according to the invention may, for example, have the following sodium concentrations: 5 to 1250 mg/ 100g or ml, 5 to 600 mg / 100 g or ml, 5 to 250 mg / 100 g or ml, 5 to 200 mg/100 g or ml, 5 to 140 mg /100 g or ml, 5 to 100 mg/100 g or ml, or 5 to 40 mg/100g or ml. While 5 mg is a useful minimum sodium concentration, if less saltiness is to be achieved, the lower concentration may be even lower, for example 4, 3, 2, or 1 mg / 100 g or 100 ml. Furthermore, if a very high degree of saltiness is to be achieved, a higher salt content may be chosen.

The NaCl concentration of consumables will be labelled "reduced", "low" or "free" in sodium or salt according to national regulations. Low salt or low sodium consumables are defined differently in different countries according to national regulations. Some examples are given below and the corresponding concentrations are also useful for consumables according to the invention. Consumables with a sodium content of no more than 40 mg /100 g or 100 ml (UK), or 120 mg/100 g or 100 ml (AU, NZ), or 140 mg /100 g or per serving (Canada, US) may be labelled low salt. "Very low sodium" consumables have 35 mg or less per serving (US). Consumables labelled as "salt reduced" must be reduced in sodium compared to the standard product by at least 90 mg/100 g or ml and must not exceed a total of 600 mg/100 g or ml (AU, NZ). "Salt free" or "sodium free" consumables may not contain more sodium than 5 mg /100 g or 100 ml (UK), or not more than 5 mg / indicated amount or serving (Canada, US).

A person skilled in the art will appreciate that formulations and consumables may contain additional ingredients which may comprise various additives and excipients well known in the art, including anti-caking agents, antifoaming agents, anti-oxidants, binders, colorants, diluents, disintegrants, emulsifiers, encapsulating agents or formulations, enzymes, fats, flavour-enhancers, flavouring agents, gums, lubricants, polysaccharides, preservatives, proteins, solubilisers, solvents, stabilisers, sugar-derivatives, surfactants, sweetening agents, vitamins, waxes, and the like. Solvents which may be used are known to those skilled in the art and include e.g. ethanol, ethylene glycol, propylene glycol, glycerin, triacetin, diethyl phthalate and dimethyl phthalate. Encapsulants and gums include maltodextrin, gum arabic, alginates, gelatin, modified starch, and polysaccharides. Examples of additives, excipients, carriers, diluents or solvents for flavour or fragrance compounds may be found e.g. in "Perfume and Flavor Materials of Natural Origin., S. Arctander, Ed., Elizabeth, N.J., 1960; in "Perfume and Flavor Chemicals", S. Arctander, Ed., Vol. I & II, Allured Publishing Corporation, Carol Stream, USA, 1994; in "Flavourings", E. Ziegler and H. Ziegler (ed.), Wiley-VCH Weinheim, 1998, and "CTFA Cosmetic Ingredient Handbook", J.M. Nikitakis (ed.),1st ed., The Cosmetic, Toiletry and Fragrance Association, Inc., Washington, 1988.

There now follows a series of non-limiting examples that serve to illustrate the invention.

### Examples

### Examples 1-8

Unless otherwise indicated, all sensory tests are triangle tests and are performed according to the guidelines in "Amtliche Sammlung von Untersuchungsverfahren nach § 35 LMBG (Lebensmittel- und Bedarfsgegenständegesetz)"; L 00.90 7 , Untersuchung von Lebensmitteln , Sensorische Prüfverfahren, Dreiecksprüfung (Übemahme der gleichnahmigen Deutschen Norm DIN ISO 4120, Ausgabe Januar 1995), as follows:

The sensory panel is trained to evaluate the taste of aqueous solutions (4 ml each) of the following standard taste compounds by using a triangle test as described in the literature (Wieser and Belitz, Z. Lebensm. Unters. Forsch., 1975, 159, 65-72): sucrose (40 mmol/L) for sweet taste; citric acid (5 mmol/L) for sour taste; NaCl (12 mmol/L) for salty taste; caffeine (2 mmol/L) for bitter taste; and monosodium glutamate (MSG; 6 mmol/L) for umami taste. For kokumi taste, a solution of glutathione (10 mmol/L) in diluted chicken broth concentrate (Goumet Bouillon Huhn, Maggi, Singen, Germany; 3 g/100 g bottled water (Evian®)) is prepared and compared to the taste of chicken broth with no glutathione added.

All sensory analyses are performed in a sensory panel room at 22-25 °C over three different sessions by a trained panel of 8 to 10 individuals.

For recording the taste profiles, samples are prepared as indicated in the examples below. Taste profiles of samples are determined in a triangle test in three different sessions. Panellists refrain from eating or drinking for at least 1 hour prior to the session. At the start of the session and before each trial, the subject rinsed with water and expectorated. The participants receive a set of two blanks and one taste sample. Liquid samples are swirled around in the mouth briefly and expectorated. Solid samples are chewed for 20 seconds and then expectorated.. After indicating, which glass vial shows a different taste profile and description of the distinction, the participant receives another trial set of two blanks and one taste sample. Each sample with additive is compared to two reference samples without additives. Kokumi intensity is rated from 0-5 according to a scale from 0 to 5 (with 5 most intensive). The additive is added to a consumable and the sample is homogenised. The samples are presented to the sensory panel directly after homogenisation. The sensory panel included 6-8 trained individuals with exception of example 4, here the panel consists of 10 trained individuals.

### Example 1a

### S-substituted peptides in sodium salt solution (30 mmol/L NaCL)

The compounds listed in the table below are dissolved in an aqueous solution (pH 6.5) of NaCl (30 mmol/L). The taste intensity of this solution is compared to NaCl solutions of increasing concentrations ranging from 30 to 100 mmol/L. The concentration of NaCl solutions showing isointensity are determined. The results are indicated in the table below.

| **sample #** | **NaCl (30 mmol/L) sample** | **concentration of an isointense NaCl-solution** |
|---|---|---|
| 1 | Reference without additive | 30 mmol/L |
| 2 | S-(α,β -dicarboxyethyl)-L-cysteine (10 mmol/L) | 40 mmol/L |
| 3 | S-(α,β -dicarboxyethyl) γ-L-glutamyl-L-cysteinyl-glycine (10 mmol/L) | 48 mmol/L |
| 4 | S-(α,β -dicarboxyethyl) γ-L-glutamyl-cysteine (10 mmol/L) | 60 mmol/L |
| 5 | β-S-(α,γ -dicarboxypropyl) γ-L-glutamyl-L-cysteinyl-glycine (10 mmol/L) | 48 mmol/L |
| 6 | S-(α,β -dicarboxyethyl) γ-L-glutamyl-cysteine ethyl ester (10 mmol/L) | 48 mmol/L |
| 7 | (S-α,β-dicarboxyethyl)-cystein ethyl ester (10 mmol/L) | 42 mmol/L |
| 8 | β-S-(carboxyethyl) γ-L-glutamyl-L-cysteinyl-glycine (10 mmol/L) | 38 mmol/L |
| 9 | (S-α,β-dicarboxyethyl)-cystein methyl ester (10 mmol/L) | 38 mmol/L |

Panelists unanimously conclude that samples 2 to 9 are more salty than the control (sample 1). With regard to isointensity, for example, the sample containing NaCl (30 mmol/L) and S-(α,β-dicarboxyethyl) γ-L-glutamyl-cysteine (10 mmol/L) shows isointenity in salty taste to a 60 mmol/L NaCl solution. This means that this sample reaches a taste sensation of similar intensity as a NaCl solution with double the amount of NaCl.

### Example 1b

### S·substituted peptides in sodium and potassium salt solution (30 mmol/L NaCl. 15 mmol/L KCI)

The salt-enhancing effect in solutions of NaCl and KCI is tested. KCI is often used in small concentrations to reduce sodium without loss of salty taste. However, KCI exhibits beside the salty taste an unpleasant metallic bitter taste which makes its taste distinct from NaCl, which provides a more "clean" salty taste without off-notes.

For the samples compared and the results see the table below.

| **sample #** | **NaCl solution (30 mmo)/L)** | **KCI (15 mmo)/L)** | **salt enhancer (10 mmol/L)** | **concentration of an isointense NaCl-solution** |
|---|---|---|---|---|
| 1 | + | - | - | 30 mmol/L |
| 7 | + | + | - | 40 mmol/L |
| 8 | + | + | S-(α,β-dicarboxyethyl) y-L-glutamyl-L-cysteinyl-glycine | 57 mmol/L |
| 9 | + | + | Arginine formate (10 mmol/L) + S-(α,β-dicarboxyethyl) y-L-glutamyl-L-cysteinyl-glycine | 88 mmol/L |

Comparison of sample 8 or 9 (both with salt enhancer) with the same solution lacking the salt enhancer (sample 7) shows an increase of saltiness. The samples with salt enhancer are perceived as an equivalent of 57 or 88 mmol/L NaCl, while without salt enhancer the intensity of only 40 mmol/L NaCl is perceived.

Panelists describe a more NaCl-like clean salty taste profile of the KCl-containing samples with salt enhancer (samples 8 and 9), which are preferred by all panellists over the sample without the salt enhancer which displays the bitter/metallic off-notes typical for KCI much more perceivably.

### Example 2

### S-(α,β-dicarboxyethyl) y-L-glutamyl-L-cysteinyl-glycine in mashed potatoes

Instant mashed potatoes (Pfanni, Unilever Bestfoods) are prepared as specified in the table below. The samples are tasted by a panel of 6 at a temperature of 60°C.

| **sample #** | **Mashed potatoes samples and additives (concentration)** | **Sensory results (compared to 1)** |
|---|---|---|
| 1 | Reference without additives | - |
| 2 | NaCl (2000 ppm) | More salty, no effect on mouthfulness, no umami taste |
| 3 | S-(α,β-dicarboxyethyl) y-L-glutamyl-L-cysteinyl-glycine (100 ppm) | Slightly more salty and broth-like (kokumi) |
| 4 | S-(α,β-dicarboxyethyl) γ-L-glutamyl-L-cysteinyl-glycine (500 ppm) | Intensely salty, broth-like (kokumi), enhanced taste and mouthfulness |

Out of a panel of 6 individuals, 5 indicate sample 3 as slightly more salty and broth-like than Sample 1. One panelist indicates that both samples are equally salty. All panellists describe sample 4 (higher concentrated compound) as intensely salty, broth-like and to give an enhanced taste and mouthfulness. Sample 2 (NaCl) is described as more salty than sample 1, but no effect on mouthfulness or umami taste is observed.

### Example 3a

### Salt enhancing compounds in chicken broth

Sensory tests (triangle test) are performed at least twice for each compound using sensory panels of different individuals to confirm results.

Chicken broth is prepared by diluting 3 g of a chicken broth concentrate (Gourmet Bouillon Huhn, Nestle) with 100 ml water. Additives are added as specified in table below in a concentration of 10 mmol/L. The pH-value of all samples is adjusted to 6.5 using formic acid (0.1 mol/L) or sodium hydroxide (0.1 mol/L).

Kokumi intensity is rated according to a scale from 0 to 5 (with 5 most intensive). GSH (10 mmol/L) is determined to have an kokumi intensity of 3.5 in all tests. The additive is added to a consumable and the sample is homogenised.

The samples are presented to the sensory panel directly after homogenisation. The sensory panel included 8 trained individuals.

The results of the tests are indicated in the table below.

| **Chicken broth samples** | **Sensory results (compared to 1)** | **Kokumi Intensity (0-5)** |
|---|---|---|
| Reference (without additives) | - | 2 |
| GSH reduced (10 mmol/L) | Increased complexity and mouthful ness, more rich, more impact, punch, long lasting taste sensation, more meaty, chicken-like | 3.5 |
| S-(α,β -dicarboxyethyl) y-L-glutamyl-L-cysteinyl-glycine (400 ppm) | Strongly increased saltiness, "oversalted soup", greater mouthfulness, long lasting kokumi taste | 3.5 |

### Example 3b

### Salt enhancing compounds in chicken broth

The samples are tested as described above in example 3 a. The results of the tests are indicated in the table below.

| **sample #** | **additive** | **Sensory results (compared to 1)** |
|---|---|---|
| 1 | Reference (without additives) | - |
| 2 | NaCl (2000 ppm) | More salty |
| 3 | MSG (2000 ppm) | Higher umami intensity |
| 4 | S-(α,β-dicarboxyethyl) γ-L-glutamyl-L-cysteinyl-glycine (400 ppm) | More salty, greater mouthfulness, long lasting kokumi taste |
| 5 | S-(α,β-dicarboxyethyl) y-L-glutamyl-L-cysteine (400 ppm) | More salty, highest salt intensity of all samples, greater mouthfulness, long lasting kokumi taste |
| 6 | S-(α,β -dicarboxyethyl) cysteine (400 ppm) | More salty |
| 7 | β-S-(α,γ -dicarboxypropyl) γ-L-glutamyl-L-cysteinyl-glycine (400 ppm) | More salty, greater mouthfulness, long lasting kokumi taste |
| 8 | β-S-(carboxyethyl) γ-L-glutamyl-L-cysteinyl-glycine (400 ppm) | More salty, greater mouthfulness, long lasting kokumi taste |

All 8 panellists rate sample 2 as being more salty than sample 1, and sample 3 as having a higher umami intensity. The remaining samples are described as more salty than control sample 1, the highest salt taste intensity is perceived for sample 5. In addition, samples 4, 5, 7 and 8 have a greater mouthfulness and provoke a long lasting kokumi taste sensation.

### Example 4

### Salt enhancing compounds in cream cheese

Cream cheese (10 g; Philadelphia, Kraft) is intimately mixed with the additive in the concentration indicated in the table below. Samples are compared by a panel of 10 trained panelists.

| **sample #** | **Cream cheese samples** | **Sensory results Compared to 1** | **Sensory results compared to 2** |
|---|---|---|---|
| 1 | Reference without additives | - | - |
| 2 | NaCl Reference (2000 ppm) | More salty | - |
| 3 | S-(α,β -dicarboxyethyl) y-L-glutamyl-L-cysteinyl-glycine (1000 ppm) | More salty, more complex, increased mouthfulness and richness | More salty, more complex, increased mouthfulness and richness |
| 4 | S-(α,β-dicarboxyethyl) γ-L-glutamyl-L-cysteine (1000 ppm) | More salty, more complex, increased mouthfulness and richness | More salty, more complex, increased mouthfulness and richness |
| 5 | S-(α,β -dicarboxyethyl)-L-cysteine (1000 ppm) | More salty | Slightly more salty |

All panelists indicate that samples 2 to 5 are more salty than sample 1. In addition, samples 3 to 5 are higher rated for saltiness than sample 2. From these results it can be seen that the addition of S-(α,β-dicarboxyethyl) γ-L-glutamyl-L-cysteinyl-glycine or S-(α,β-dicarboxyethyl) γ-L-glutamyl-cysteine in a concentration of 1000 ppm has a greater effect on saltiness than the addition of NaCl in a concentration of 2000 ppm. Furthermore, samples 3 and 4 are described as more complex and having an increased mouthfulness and richness.

### Example 5

### Salt enhancing compounds in ketchup

S-(α,β-dicarboxyethyl) γ-L-glutamyl-cysteine or S-(α,β-dicarboxyethyl) γ-L-glutamyl-L-cysteinyl-glycine are added to ketchup in the concentrations indicated below. The results are shown in the table below.

| **sample #** | **Ketchup samples** | **Sensory results compared to 1** |
|---|---|---|
| 1 | Reference without additive | - |
| 2 | S-(α,β-dicarboxyethyl) y-L-glutamyl-L-cysteinyl-glycine (500 ppm) | Slightly more salty |
| 3 | S-(α,β-dicarboxyethyl) γ-L-glutamyl-L-cysteinyl-glycine (1000 ppm) | More salty. More intense taste. |
| 4 | S-(α,γ-dicarboxyethyl) y-L-glutamyl-L-cysteinyl-glycine (5000 ppm) | More salty. More intense taste. |
| 5 | S-(α,β-dicarboxyethyl) y-L-glutamyl-L-cysteine (500 ppm) | More salty. More intense taste. |

Sample 2 is indicated to be preferred by 7 out of 8 panelists, and described as slightly more salty and having a longer lasting, more intense taste. One panelist detects no difference between samples 2 and 1.

All panelists find samples 3, 4, and 5 more salty than 1 and 2, and of more intense taste.

### Example 6

### Salt enhancing compounds in whipped cream

Cream is mixed with S-(α-β-dicarboxyethyl) y-L-glutamyl-L-cysteinyl-glycine or S-(α,β-dicarboxyethyl) y-L-glutamyl-cysteine, whipped and compared to the reference. The results are shown in the table below.

| **sample #** | **Whipped cream sample** | **Sensory results compared to 1** |
|---|---|---|
| 1 | Reference without additive | - |
| 2 | S-(α,β-dicarboxyethyl) γ-L-glutamyl-L-cysteinyl-glycine (500 ppm) | Higher taste intensity, strong increase of saltiness and mouthfulness. |
| 3 | S-(α-β-dicarboxyethyl) γ-L-glutamyl-L-cysteine (500 ppm) | Higher taste intensity, strong increase of saltiness and mouthfulness. Effect is stronger when compared to sample 2. |

Panelists find samples 2 and 3 have a higher intensity, and the difference is described as a strong increase of saltiness and mouthfulness. For sample 3 this effect is found to be greater than for sample 2.

### Example 7

### Salt enhancing compounds in soft cheese

The salt enhancing compounds are added to soft cheese and the samples are stirred to homogenity. Samples are compared to a reference. The results are indicated in the table below.

| **sample #** | **Soft cheese sample** | **Sensory results compared to 1** |
|---|---|---|
| 1 | Reference without additive | - |
| 2 | S-(α,β-dicarboxyethyl) y-L-glutamyl-L-cysteinyl-glycine (500 ppm) | Preferred. Strongly increased saltiness. |
| 3 | S-(α-β-dicarboxyethyl) y-L-glutamyl-L-cysteine (500 ppm) | Preferred. Strongly increased saltiness. |

7 out of 8 panelists indicate sample 2 to be preferred. The difference is described as a strong increase in saltiness. All 8 panelists detect a significant increase in saltiness in sample 3.

### Example 8

### Synthesis of S-substituted cysteine and cysteine containing peptides

The reaction between a thiol compound (cysteine (H-Cys-OH), glutathione (H-γ-Glu-Cys-Glγ-OH), y-L-glutamyl-cysteine (H-γ-Glu-Cys-OH), H-ß-Asp-Cys-Gly-OH, ß-L-Asp-Cys-OH), and an unsaturated carbonyl compound having at least one double bond (maleic acid, fumaric acid, glutaconic acid, acrylic acid, 2-pentenoic acid, aconitic acid etc.) is performed according to the procedure reported by Morgan and Friedmann (Biochemical Journal; 32 (1938); 733-742) as follows:

The unsaturated carbonyl (10 mmol) and the thiol compound (10 mmol) are dissolved in water (100 ml), pH is adjusted to pH 7.4 with NaOH (1 mol/L) and incubated at 37 °C for 24 h.

After freeze-drying, the desired compounds are purified by means of gel permeation chromatographie using Sephadex G-10 (Amersham Bioscience, Uppsalla, Sweden) as stationary phase and water as mobile phase. The desired compounds elute after 800 mL of mobile phase, the identity and purity is confirmed by means of LC-MS and NMR spectroscopy.

## Claims

1. Use as a flavour or salt enhancer of at least one compound according to the formula (I), or a salt thereof, 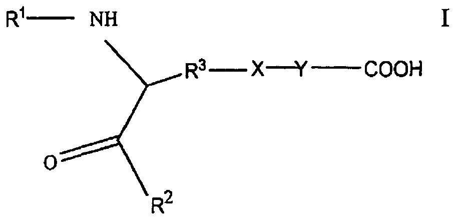 wherein the residues R¹, R², R³, X and Y are selected as follows:
R¹ is a residue selected from the group of H, an aminoacid linked via a peptide bond selected from the group consisting of γ-Glu, α-Glu, ß-Asp, α-Asp, β-Ala, α-Ala, α-Val, α-Leu, α-Ile, α-Met, α-Pro, α-Phe, α-Trp, α-Ser, α-Thr, α-Asn, α-Gln, α-Tyr, α-Cys, α-Lys, α-Arg, α-His, α-Asp, α-Glu, gamma amino butyric acid (GABA), and an uncommon amino acid including 4-hydroxyprolin, ε-N,N,N-trimethyllysine, 3-methylhistidine, 5-hydroxylysine, O-phosphoserine, gamma-carboxyglutamate, ε-N-acetyllysine, ω-N-methylarginine, N-acetylserine, N,N,N-trimethylalanine, N-formylmethionine;
R² is a residue selected from the group consisting of OH, a C₁-C₅linear or branched alkoxy residue including -O-CH₃, -O-CH₂-CH₃, -O-CH₂CH₂CH₃, -O-CH(CH₃)CH₃, -O-CH₂CH(CH₃)₂, -O-CH₂CH(CH₃)(CH₂CH₃), and -O-CH₂CH₂CH(CH₃)₂, and an aminoacid linked via a peptide bond selected from the group consisting of Gly, β-Ala, α-Ala, α-Val, α-Leu, α-Ile, α-Met, α-Pro, α-Phe, α-Trp, α-Ser, α-Thr, α-Asn, α-Gln, α-Tyr, α-Cys, α-Lys, α-Arg, α-His, α-Asp, a-Glu, gamma amino butyric acid (GABA), and an uncommon amino acid including 4-hydroxyprolin, ε-N,N,N-trimethyllysine, 3-methylhistindine, 5-hydroxylysine, O-phosphoserine, gamma-carboxyglutamate, ε-N-acetyllysine, ω-N-methylarginine, N-acetylserine, N,N,N-trimethylalanine, N-formylmethionine;
R³ is a residue selected from -CH₂- or -CH₂CH₂-;
X is a residue selected from -S- or -O-; and
Y is a residue selected from the group consisting of -CH(COOH)-, -C(COOH)₂-, -C(CH₂COOH)₂-, -C(CH₂COOH)(COOH)-, -C(CH(COOH)₂)(COOH)-, - CH(CH₂COOH)-, -CH(CH₂CH₂COOH)-, -CH(CH₂CH₂CH₂COOH)-, -CH(CH(COOH)₂)-, - CH(CH(COOH)CH(COOH)₂)-, -CH(CH₂CH(COOH)₂)-, -CH(CH(COOH)CH₂COOH)-, -CH₂-CH(COOH)-, -CH₂-C(COOH)₂-, -CH₂-C(CH₂COOH)₂-, -CH₂-C(CH₂COOH)(COOH)-, -CH₂-C(CH(COOH)₂)(COOH)-, -CH₂-CH(CH₂COOH)-, -CH₂-CH(CH₂CH₂COOH)-, -CH₂-CH(CH₂CH₂CH₂COOH)-, -CH₂-CH(CH(COOH)₂)-, -CH₂-CH(CH(COOH)CH(COOH)₂)-, -CH₂-CH(CH₂CH(COOH)₂)-, -CH₂-CH(CH(COOH)CH₂COOH)-, -CH(COOH)-CH₂-, -C(COOH)₂-CH₂-, -C(CH₂COOH)₂-CH₂-, -C(CH₂COOH)(COOH)-CH₂-, - C(CH(COOH)₂)(COOH)-CH₂-, -CH(CH₂COOH)-CH₂-, -CH(CH₂CH₂COOH)-CH₂-, -CH(CH₂CH₂CH₂COOH)-CH₂-, - CH(CH(COOH)₂)-CH₂-, -CH(CH(COOH)CH(COOH)₂)-CH₂-, -CH(CH₂CH(COOH)₂)-CH₂-, - CH(CH(COOH)CH₂COOH)-CH₂-.

2. Use according to claim 1 wherein R¹ is selected from the group consisting of γ-Glu or (ß-Asp.

3. Use according to claim 1 wherein R₂ is a residue selected from the group consisting of OH, Gly , and ß-Ala.

4. Use according to claim 1 wherein R₂ is a C₁-C₅ linear or branched alkoxy residue including -O-CH₃, -O-CH₂-CH₃, -O-CH₂CH₂CH₃, -O-CH(CH₃)CH₃, -O-CH₂CH(CH₃)₂, -O-CH₂CH(CH₃)(CH₂CH₃), and -O-CH₂CH₂CH(CH₃)₂.

5. Use according to claim 1 wherein R¹ is γ-Glu, R₂ is a residue selected from the group consisting of OH, Gly, and β-Ala, and R³ is a residue selected from CH₂ and CH₂CH₂; X is a residue selected from S and O; and Y is a residue selected from the group consisting of CH(COOH), CH(COOH)CH₂, CH(CH₂COOH)CH₂, and CH(CH₂CH₂COOH).

6. Use according to claim 1 wherein R¹ is γ-Glu, R₂ is a C₁-C₅ linear or branched alkoxy residue including -O-CH₃, -O-CH₂-CH₃. -O-CH₂CH₂CH₃, -O-CH(CH₃)CH₃, -O-CH₂CH(CH₃)₂, -O-CH₂CH(CH₃)(CH₂CH₃), and -O-CH₂CH₂CH(CH₃)₂, and R³ is a residue selected from CH₂ and CH₂CH₂; X is a residue selected from S and O; and Y is a residue selected from the group consisting of CH(COOH), CH(COOH)CH₂, CH(CH₂COOH)CH₂, and CH(CH₂CH₂COOH).

7. Use according to any one of claims 1 to 6 wherein X is S.

8. Use according to any one of claims 1 to 6 wherein R³ is CH₂.

9. Use according to any one of claims 1 to 6 wherein Y is CH(COOH), CH(COOH)CH_{2,} CH(CH₂COOH)CH₂, and CH(CH₂CH₂COOH).

10. Use according to any one of claims 1 to 6 wherein Y is -CH₂-, -CH(COOH)-, -CH(COOH)CH₂-, - CH(CH(COOH)₂)-, -CH₂CH₂-, -CH₂CH₂ CH₂-, and -CH(CH₂COOH)CH₂.

11. A compound of formula I as defined in any one of claims 1 to 10 with the proviso that the compound is not selected from the group consisting of S-(α,β-dicarboxyethyl) y-L-glutamyl-L-cysteinyl-glycine, S-(α,β-dicarboxyethyl) cysteine, 3-(carboxymethoxy)-alanine, S-carboxymethyl-glutathione (glutaramic acid), S-carboxymethyl-cysteinyl-glycine, (S-carboxymethyl)- lysyl -cysteine, S-dicarboxymethyl-glutathione, S-carboxymethyl-cysteine, S-(1,2-dicarboxyethyl)-glutathione, and S-(1,2-dicarboxyethyl)-cysteine.

12. A method to form a *γ-glutamyl dipeptide* compound of formula I as defined in any one of claims 1 to 10 by enzymatical synthesis *using gamma-glutamyl-transpeptidase,* with the proviso that the compound is not selected from the group consisting of S-(α,β-dicarboxyethyl) γ-L-glutamyl-L-cysteinyl-glycine, S-(α,β-dicarboxyethyl) cysteine, 3-(carboxymethoxy)-alanine, S-carboxymethyl-glutathione (glutaramic acid), S-carboxymethyl-cysteinyl-glycine, (S-carboxymethyl)- lysyl-cysteine, S-dicarboxymethyl-glutathione, S-carboxymethyl-cysteine, S-(1,2-dicarboxyethyl)-glutathione, and S-(1,2-dicarboxyethyl)-cysteine.

13. A flavour composition for enhancing the salt taste of food products, beverages, and oral care products, comprising at least one compound of formula I as defined in any one of claims 1 to 10, wherein the flavour composition comprises at least one excipient selected from the group consisting of a flavouring agent and a flavour enhancer.

14. Composition according to claim 13 comprising a compound selected from the group consisting of arginine aspartate and arginine formate in a ratio of 10:1 to 1:10.

15. A flavour composition according to claim 13 wherein the compounds of formula I are present in form of a crude or purified extract selected from the group consisting of a enzyme extract, a plant extract, a fermentation extract, a cell culture fermentation extract, a bacteria fermentation extract, a fungi fermentation extract, and a yeast fermentation extract.

16. A consumable, selected from the group consisting of food products, beverages, and oral care products, comprising a compound of formula I defined in any one of claims 1 to 10.

17. A method for enhancing the saltiness of a consumable, comprising the addition of a compound as defined in any one of claims 1 to 10, or a composition as defined in claims 13 to 15, and an amount of salt of at least 5 mmol/kg.

## Patentansprüche

1. Verwendung wenigstens einer Verbindung gemäß Formel (I) oder eines Salzes davon als Geschmacks-oder Salzverstärker, 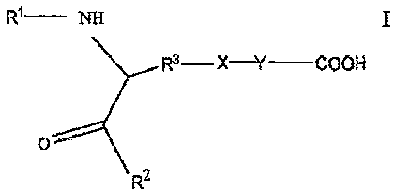 wobei die Reste R¹, R², R³, X und Y folgendermaßen ausgewählt sind:
R¹ ist ein Rest, ausgewählt aus der Gruppe von H, einer über eine Peptidbindung gebundenen Aminosäure, ausgewählt aus der Gruppe bestehend aus γ-Glu, α-Glu, β-Asp, α-Asp, β-Ala, α-Ala, α-Val, α-Leu, α-Ile, α-Met, α-Pro, α-Phe, α-Trp, α-Ser, α-Thr, α-Asn, α-Gln, α-Tyr, α-Cys, α-Lys, α-Arg, α-His, α-Asp, α-Glu, gamma-Aminobuttersäure (GABA) und einer ungewöhnlichen Aminosäure, einschließlich 4-Hydroxyprolin, ε-N,N,N-Trimethyllysin, 3-Methylhistidin, 5-Hydroxylysin, O-Phosphoserin, gamma-Carboxyglutamat, ε-N-Acetyllysin, ω-N-Methylarginin, N-Acetylserin, N,N,N-Trimethylalanin, N-Formylmethionin;
R² ist ein Rest, ausgewählt aus der Gruppe bestehend aus OH, einem linearen oder verzweigten C₁-C₅-Alkoxyrest, einschließlich -O-CH₃, -O-CH₂-CH₃, -O-CH_{2C}H₂CH₃, -O-CH(CH₃)CH₃, -O-CH₂CH (CH₃)2, -0-CH₂CH (CH₃) (CH₂CH₃) und -O-CH₂CH₂CH (CH₃)₂, und einer über eine Peptidbindung gebundenen Aminosäure, ausgewählt aus der Gruppe bestehend aus Gly, β-Ala, α-Ala, α-Val, α-Leu, α-Ile, α-Met, α-Pro, α-Phe, α-Trp, α-Ser, α-Thr, α-Asn, α-Gln, α-Tyr, α-Cys, α-Lys, α-Arg, α-His, α-Asp, α-Glu, gamma-Aminobuttersäure (GABA) und einer ungewöhnlichen Aminosäure, einschließlich 4-Hydroxyprolin, ε-N,N,N-Trimethyllysin, 3-Methylhistindin, 5-Hydroxylysin, O-Phosphoserin, gamma-Carboxyglutamat, ε-N-Acetyllysin, ω-N-Methylarginin, N-Acetylserin, N,N,N-Trimethylalanin, N-Formylmethionin;
R³ ist ein Rest, ausgewählt aus -CH₂- und -CH₂CH₂-;
X ist ein Rest, ausgewählt aus -S- und -0-; und
Y ist ein Rest, ausgewählt aus der Gruppe bestehend aus -CH(COOH)-, -C(COOH)₂-, -C(CH₂COOH)₂-, -C(CH₂COOH) (COOH)-, -C(CH(COOH)₂) (COOH) -, -CH (CH₂COOH) -, -CH (CH₂CH₂COOH) -, -CH (CH₂CH₂CH₂COOH) -, -CH(CH(COOH)₂)-, -CH(CH(COOH)CH(COOH)₂)-, -CH (CH₂CH(COOH)₂) -, -CH (CH (COOH) CH₂COOH) -, -CH₂-CH(COOH)-, -CH₂-C(COOH)₂-, -CH₂-C (CH₂COOH)₂-, -CH₂-C(CH₂COOH) (COOH)-, -CH₂-C(CH(COOH)₂) (COOH)-, -CH₂-CH(CH₂COOH)-, -CH₂-CH (CH₂CH₂COOH)-, -CH₂-CH (CH₂CH₂CH₂COOH)-, -CH₂-CH (CH (COOH)₂)-, -CH₂-CH(CH(COOH)CH(COOH)₂)-, -CH₂-CH (CH₂CH (COOH)₂) -, -CH₂-CH(CH(COOH)CH2COOH)-, -CH(COOH)-CH₂-, -C (COOH)₂-CH₂-, -C (CH₂COOH)₂-CH₂-, -C (CH₂COOH) (COOH)-CH₂-, -C(CH(COOH)₂) (COOH)-CH₂-, -CH (CH₂COOH) -CH₂-, -CH (CH₂CH₂COOH) -CH₂-, -CH (CH₂CH₂CH₂COOH) -CH₂-, -CH (CH (COOH)₂) -CH₂-, -CH (CH (COOH) CH (COOH)₂) -CH₂-, -CH (CH₂CH (COOH)₂) -CH₂-, -CH(CH(COOH)CH₂COOH)-CH₂-.

2. Verwendung gemäß Anspruch 1, wobei R¹ ausgewählt ist aus der Gruppe bestehend aus γ-Glu und β-Asp.

3. Verwendung gemäß Anspruch 1, wobei R₂ ein Rest ist, ausgewählt aus der Gruppe bestehend aus OH, Gly und β-Ala.

4. Verwendung gemäß Anspruch 1, wobei R₂ ein linearer oder verzweigter C₁-C₅-Alkoxyrest ist, einschließlich -O-CH₃, -O-CH₂CH₃, -O-CH₂CH₂CH₃, -0-CH(CH₃)CH₃, -O-CH₂CH (CH₃)₂, -O-CH₂CH (CH₃) (CH₂CH₃) und -O-CH₂CH₂CH (CH₃)₂.

5. Verwendung gemäß Anspruch 1, wobei R¹ γ-Glu ist, R₂ ein Rest ist, ausgewählt aus der Gruppe bestehend aus OH, Gly und β-Ala, und R³ ein Rest ist, ausgewählt aus CH₂ und CH₂CH₂; X ein Rest ist, ausgewählt aus S und 0; und Y ein Rest ist, ausgewählt aus der Gruppe bestehend aus CH(COOH), CH(COOH)CH₂, CH(CH₂COOH)CH₂ und CH(CH₂CH₂COOH).

6. Verwendung gemäß Anspruch 1, wobei R¹ γ-Glu ist, R₂ ein linearer oder verzweigter C₁-C₅-Alkoxyrest ist, einschließlich -O-CH₃, -O-CH₂-CH₃, -O-CH₂CH₂CH₃, -0-CH(CH₃)CH₃, -O-CH₂CH (CH₃)₂, -O-CH₂CH (CH₃) (CH₂CH₃) und -O-CH₂CH₂CH (CH₃)₂, und R³ ein Rest ist, ausgewählt aus CH₂ und CH₂CH₂; X ein Rest ist, ausgewählt aus S und 0; und Y ein Rest ist, ausgewählt aus der Gruppe bestehend aus CH(COOH), CH(COOH)CH₂, CH(CH₂COOH)CH₂ und CH(CH₂CH₂COOH).

7. Verwendung gemäß einem der Ansprüche 1 bis 6, wobei X S ist.

8. Verwendung gemäß einem der Ansprüche 1 bis 6, wobei R³ CH₂ ist.

9. Verwendung gemäß einem der Ansprüche 1 bis 6, wobei Y CH(COOH), CH(COOH)CH₂, CH (CH₂COOH) CH₂ oder CH(CH₂CH₂COOH) ist.

10. Verwendung gemäß einem der Ansprüche 1 bis 6, wobei Y -CH₂-, -CH(COOH)-, -CH(COOH)CH₂-, -CH(CH(COOH)₂)-, -CH₂CH₂-, -CH₂CH₂CH₂- oder -CH(CH₂COOH)CH₂ ist.

11. Verbindung der Formel I, wie definiert in einem der Ansprüche 1 bis 10, mit der Maßgabe, dass die Verbindung nicht ausgewählt ist aus der Gruppe bestehend aus S-(α,β-Dicarboxyethyl)-γ-L-glutamyl-L-cysteinylglycin, S-(α,β-Dicarboxyethyl)cystein, 3-(Carboxymethoxy)alanin, S-Carboxymethylglutathion (Glutaramsäure), S-Carboxymethylcysteinylglycin, (S-Carboxymethyl)lysylcystein, S-Dicarboxymethylglutathion, S-Carboxymethylcystein, S-(1,2-Dicarboxyethyl)glutathion und S-(1,2-Dicarboxyethyl)cystein.

12. Verfahren zum Herstellen einer γ-Glutamyldipeptidverbindung der Formel I, wie definiert in einem der Ansprüche 1 bis 10, durch enzymatische Synthese unter Verwendung von gamma-Glutamyltranspeptidase, mit der Maßgabe, dass die Verbindung nicht ausgewählt ist aus der Gruppe bestehend aus S-(α,β-Dicarboxyethyl)-γ-L-glutamyl-L-cysteinylglycin, S-(α,β-Dicarboxyethyl)cystein, 3-(Carboxymethoxy)alanin, S-Carboxymethylglutathion (Glutaramsäure), S-Carboxymethylcysteinylglycin, (S-Carboxymethyl)lysylcystein, S-Dicarboxymethylglutathion, S-Carboxymethylcystein, S-(1,2-Dicarboxyethyl)glutathion und S-(1,2-Dicarboxyethyl)cystein.

13. Aromazusammensetzung zum Verstärken des Salzgeschmacks von Nahrungsmittelprodukten, Getränken und Mundpflegeprodukten, umfassend wenigstens eine Verbindung der Formel I, wie definiert in einem der Ansprüche 1 bis 10, wobei die Aromazusammensetzung wenigstens einen Exzipienten, ausgewählt aus der Gruppe bestehend aus einem Aromastoff und einem Geschmacksverstärker, umfasst.

14. Zusammensetzung gemäß Anspruch 13, umfassend eine Verbindung, ausgewählt aus der Gruppe bestehend aus Argininaspartat und Argininformiat in einem Verhältnis von 10:1 bis 1:10.

15. Aromazusammensetzung gemäß Anspruch 13, wobei die Verbindungen der Formel I in der Form eines rohen oder gereinigten Extrakts, ausgewählt aus der Gruppe bestehend aus einem Enzymextrakt, einem Pflanzenextrakt, einem Fermentationsextrakt, einem Zellkultur-Fermentationsextrakt, einem Bakterienfermentationsextrakt, einem Pilzfermentationsextrakt und einem Hefefermentationsextrakt, vorliegen.

16. Verbrauchsgut, ausgewählt aus der Gruppe bestehend aus Nahrungsmittelprodukten, Getränken und Mundpflegeprodukten, umfassend eine Verbindung der Formel I, wie definiert in einem der Ansprüche 1 bis 10.

17. Verfahren zum Verstärken der Salzigkeit eines Verbrauchsguts, umfassend das Zugeben einer Verbindung, wie definiert in einem der Ansprüche 1 bis 10, oder einer Zusammensetzung, wie definiert in den Ansprüchen 13 bis 15, und einer Menge an Salz von wenigstens 5 mmol/kg.

## Revendications

1. Utilisation comme arôme ou exhausteur de goût salé d'au moins un composé répondant à la formule (I), ou de l'un de ses sels, 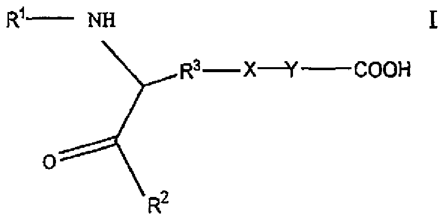 où les résidus R¹, R², R³, X et Y sont choisis de la façon suivants :
R¹ est un résidu choisi dans le groupe constitué par H, un acide aminé lié par une liaison peptidique et choisi dans le groupe constitué par γ-Glu, α-Glu, β-Asp, α-Asp, β-Ala, α-Ala, α-Val, α-Leu, α-Ile, α-Met, α-Pro, α-Phe, α-Trp, α-Ser, α-Thr, α-Asn, α-Gln, α-Tyr, α-Cys, α-Lys, α-Arg, α-His, α-Asp, α-Glu, l'acide gamma-aminobutyrique (GABA), et un acide aminé non courant, y compris les suivants : 4-hydroxyproline, ε-N,N,N-triméthyllysine, 3-méthylhistidine, 5-hydroxylysine, 0-phosphosérine, gamma-carboxyglutamate, ε-N-acétyllysine, ω-N-méthylarginine, N-acétylsérine, N,N,N-triméthylalanine, N-formylméthionine ;
R² est un résidu choisi dans le groupe constitué par OH, un résidu alkoxy en C₁-C₅ linéaire ou ramifié incluant - O-CH₃, -O-CH₂-CH₃, -O-CH₂CH₂CH₃, -O-CH(CH₃)CH₃, -O-CH₂CH(CH₃)₂, -O-CH₂CH (CH₃) (CH₂CH₃) et -O-CH₂CH₂CH (CH₃)₂, et un acide aminé lié par une liaison peptidique et choisi dans le groupe constitué par Gly, β-Ala, α-Ala, α-Val, α-Leu, α-Ile, α-Met, α-Pro, α-Phe, α-Trp, α-Ser, α-Thr, α-Asn, α-Gln, α-Tyr, α-Cys, α-Lys, α-Arg, α-His, α-Asp, α-Glu, l'acide gamma-aminobutyrique (GABA), et un acide aminé non courant, y compris les suivants : 4-hydroxyproline, ε-N,N,N-triméthyllysine, 3-méthylhistindine, 5-hydroxylysine, O-phosphosérine, gamma-carboxyglutamate, ε-N-acétyllysine, ω-N-méthylarginine, N-acétylsérine, N,N,N-triméthylalanine, N-formylméthionine ;
R³ est un résidu choisi parmi -CH₂- ou -CH₂CH₂- ;
X est un résidu choisi parmi -S- ou -0- ; et
Y est un résidu choisi dans le groupe constitué par -CH(COOH)-, -C(COOH)₂-, -C(CH₂COOH)₂-, -C(CH₂COOH) (COOH)-, -C(CH(COOH)₂) (COOH)-, -CH(CH₂COOH)-, -CH(CH₂CH₂COOH)-, -CH(CH₂CH₂CH₂COOH)-, -CH(CH(COOH)₂)-, - CH(CH(COOH)CH(COOH)₂)-, -CH(CH₂CH(COOH)₂)-, - CH(CH(COOH)CH₂COOH)-, -CH₂-CH(COOH)-, -CH₂-C(COOH)₂-, - CH₂-C (CH₂COOH)₂, -CH₂-C (CH₂COOH) (COOH)-, -CH₂-C(CH(COOH)₂) (COOH)-, -CH₂-CH(CH₂COOH)-, -CH₂-CH(CH₂CH₂COOH)-, -CH₂-CH (CH₂CH₂CH₂COOH) -, -CH₂-CH(CH(COOH)₂)-, -CH₂CH (CH (COOH) CH (COOH)₂) -CH₂-CH(CH₂CH(COOH)₂)-, -CH₂-CH(CH(COOH)CH₂COOH)-, -CH(COOH)-CH₂, -C (COOH)₂-CH₂-, -C (CH₂COOH)₂-CH₂-, -C (CH₂COOH) (COOH)-CH₂-, -C(CH(COOH)₂) (COOH)-CH₂-, -CH (CH₂COOH) -CH₂-, - CH (CH₂CH₂COOH) -CH₂-, -CH (CH₂CH₂CH₂COOH) -CH₂- , - CH (CH (COOH)₂)-CH₂-, -CH (CH (COOH) CH (COOH)₂)-CH₂-, - CH(CH₂CH (COOH)₂)-CH₂-, -CH (CH (COOH) CH₂COOH)-CH₂-.

2. Utilisation selon la revendication 1, où R¹ est choisi dans le groupe constitué par γ-Glu et β-Asp.

3. Utilisation selon la revendication 1, où R₂ est un résidu choisi dans le groupe constitué par OH, Gly et β-Ala.

4. Utilisation selon la revendication 1, où R₂ représente un résidu alkoxy en C₁-C₅ linéaire ou ramifié, y compris -O-CH₃, -O-CH₂-CH₃, -O-CH₂CH₂CH₃, -0-CH(CH₃)CH₃, -O-CH₂CH (CH₃)₂, -O-CH₂CH (CH₃) (CH₂CH₃) et - OCH₂CH₂CH(CH3)₂.

5. Utilisation selon la revendication 1, où R¹ représente γ-Glu, R₂ est un résidu choisi dans le groupe constitué par OH, Gly et β-Ala, et R³ est un résidu choisi parmi CH₂ et CH₂CH₂ ; X est un résidu choisi parmi S et O ; et Y est un résidu choisi dans le groupe constitué par CH(COOH), CH(COOH)CH₂, CH(CH₂COOH)CH₂ et CH(CH₂CH₂COOH).

6. Utilisation selon la revendication 1, où R¹ représente γ-Glu, R₂ représente un résidu alkoxy en C₁-C₅ linéaire ou ramifié, y compris -O-CH₃, -O-CH₂-CH₃, -0-CH₂CH₂CH₃, -O-CH(CH₃)CH₃, -O-CH₂CH (CH₃)₂, -O-CH₂CH(CH₃) (CH₂CH₃) et -O-CH₂CH₂CH (CH₃)₂, et R³ est un résidu choisi parmi CH₂ et CH₂CH₂ ; X est un résidu choisi parmi S et O ; et Y est un résidu choisi dans le groupe constitué par CH(COOH), CH(COOH)CH₂, CH(CH₂COOH)CH₂ et CH(CH₂CH₂COOH).

7. Utilisation selon l'une quelconque des revendications 1 à 6, où X représente S.

8. Utilisation selon l'une quelconque des revendications 1 à 6, où R³ représente CH₂.

9. Utilisation selon l'une quelconque des revendications 1 à 6, où Y représente CH(COOH), CH(COOH)CH₂, CH(CH₂COOH)CH₂ ou CH(CH₂CH₂COOH).

10. Utilisation selon l'une quelconque des revendications 1 à 6, où Y représente -CH₂-, -CH(COOH)-, -CH(COOH)CH₂-, -CH(CH(COOH)₂)-, -CH₂CH₂-, -CH₂CH₂CH₂- ou - CH (CH₂COOH) CH₂.

11. Composé de formule I selon l'une quelconque des revendications 1 à 10, à condition que le composé ne soit pas choisi dans le groupe constitué par les suivants : S-(α,β-dicarboxyéthyl)γ-L-glutamyl-L-cystéinyl-glycine, S-(α,β-dicarboxyéthyl)cystéine, 3-(carboxyméthoxy)-alanine, S-carboxyméthyl-glutathione (acide glutaramique), S-carboxyméthyl-cystéinylglycine, (S-carboxyméthyl)-lysyl-cystéine, S-dicarboxyméthyl-glutathione, S-carboxyméthyl-cystéine, S-(1,2-dicarboxyéthyl)-glutathione et S-(1,2-dicarboxyéthyl)-cystéine.

12. Méthode de synthèse d'un *y-glutamyldipeptide* de formule I selon l'une quelconque des revendications 1 à 10 par synthèse enzymatique *en utilisant la gamma-glutamyl-transpeptidase,* à condition que le composé ne soit pas choisi dans le groupe constitué par les suivants : S-(α,β-dicarboxyéthyl)γ-L-glutamyl-L-cystéinyl-glycine, S-(α,γ-dicarboxyéthyl)cystéine, 3-(carboxyméthoxy)-alanine, S-carboxyméthyl-glutathione (acide glutaramique), S-carboxyméthyl-cystéinylglycine, (S-carboxyméthyl)-lysyl-cystéine, S-dicarboxyméthyl-glutathione, S-carboxyméthyl-cystéine, S-(1,2-dicarboxyéthyl)-glutathione et S-(1,2-dicarboxyéthyl)-cystéine.

13. Composition aromatique destinée à améliorer le goût salé de produits alimentaires, de boissons et de produits de soins buccaux, comprenant au moins un composé de formule I selon l'une quelconque des revendications 1 à 10, où la composition aromatique comprend au moins un excipient choisi dans le groupe constitué par un arôme et un exhausteur de goût.

14. Composition selon la revendication 13 comprenant un composé choisi dans le groupe constitué par l'aspartate d'arginine et le formiate d'arginine dans un rapport compris entre 10:1 et 1:10.

15. Composition aromatique selon la revendication 13, où les composés de formule I sont présents sous la forme d'un extrait brut ou purifié choisi dans le groupe constitué par un extrait enzymatique, un extrait végétal, un extrait de fermentation, un extrait de fermentation de culture cellulaire, un extrait de fermentation de bactéries, un extrait de fermentation de champignons et un extrait de fermentation de levures.

16. Produit consommable choisi dans le groupe constitué par les produits alimentaires, les boissons et les produits de soins buccaux, comprenant un composé de formule I selon l'une quelconque des revendications 1 à 10.

17. Procédé d'amélioration du goût salé d'un produit consommable, ledit procédé comprenant l'ajout d'un composé selon l'une quelconque des revendications 1 à 10, ou d'une composition conforme aux revendications 13 à 15, et d'une quantité de sel d'au moins 5 mmol/kg.
